# EUROPEAN PATENT SPECIFICATION

(11) **EP 4 029 433 B1**
(45) Date of publication and mention of the grant of the patent: **06.03.2024**
(21) Application number: 21151982.2
(22) Date of filing: 18.01.2021
(51) Int. Cl.: A61B 5/00, A61B 5/03

(54) **METHOD OF MONITORING INTAKE OF BREAST MILK BY AN INFANT**
VERFAHREN ZUR ÜBERWACHUNG DER AUFNAHME VON MUTTERMILCH DURCH EINEN SÄUGLING
MÉTHODE DE CONTRÔLE DE L'INGESTION DE LAIT MATERNEL PAR UN NOURRISSON

(43) Date of publication of application: 20.07.2022
(73) Proprietor: Büyükyildiz, Volkan Görkem, 70839 Gerlingen (DE)
(72) Inventor: Büyükyildiz, Volkan Görkem, 70839 Gerlingen (DE)
(74) Representative: Meyer zu Bexten, Elmar

(56) References cited:
- WO-A1-01/54488
- CN-A- 110 338 757
- US-A1- 2019 125 244

## Description

### Technical Field

The invention relates to the products and process as per the preamble of the independent claims.

### Background Art

The American Academy of Pediatrics (AAP) recommends exclusive breastfeeding for about the first six months of life, with continued breastfeeding through twelve months and beyond as appropriate complementary foods are introduced. The World Health Organization (WHO) similarly recommends that infants be exclusively breastfed for the first six months, with breastfeeding continuing for two or more years as safe and nutritionally adequate complementary foods are added.

NPL1 identifies perceived insufficient milk (PIM) as a major barrier to successful breastfeeding, that is, the belief of the nursing mother that her milk be insufficient for her infant's needs. State-of-the-art methods for mitigating PIM encompass reinforcing appropriate early infant feeding routines and closely monitoring breastfeeding dyads during the first postpartum weeks to ensure that breastfeeding is well established.

In terms of technical measures, frequent infant weight checks and periodic test weighing (comparing pre-nursing to post-nursing weight) have been proposed to provide concrete evidence for breastfeeding mothers concerned about the adequacy of milk intake by their nursling. Alternatively, breast pumps - such as disclosed in PTL1 - may be employed to extract and direct the milk into a container where its volume can be easily measured prior to bottle-feeding.

PTL2 discloses the subject matter of the preamble of Claim 1.

An apparatus for determining the amount of human milk supplied to a feeding baby during a breast-feeding session is disclosed in PTL3. The apparatus comprises a feeding cap adapted to be mounted on the nipple region of a breast of a mother. The cap defines an outlet through which milk passes to the feeding baby. A flowmeter is provided to measure the amount of milk passing through the outlet.

PTL4 discloses reflection spectroscopy to determine milk intake in the stomach.

### Summary of invention

The present invention enables percutaneous determination of breast milk intake during breastfeeding using an optical method.

### Technical Problem

While a comparison of pre-nursing to post-nursing weight of the breastfed child tends to yield imprecise results, the extraction of breast milk into a container may impair some of the benefits commonly associated with breastfeeding, for example, improved maternal bonding as well as oral and cognitive development of the infant. Both methods are considered laborious and time-consuming.

### Solution to Problem

The problem is solved as per the characterizing portion of Claim 1.

### Advantageous effect of invention

The present invention addresses the need for a rapid and reliable measurement method whereby the amount of milk consumed during a breastfeeding session can be determined conveniently and non-invasively. Consequently, any nutritional deficiencies on the part of the nursling may be detected and compensated without delay.

### Brief description of drawings

Figure 1 is a perspective view of an infant being breastfed.
Figure 2 is a longitudinal section of a collar worn around the infant's neck.
Figure 3 is a cross section of the collar.

### Description of embodiments

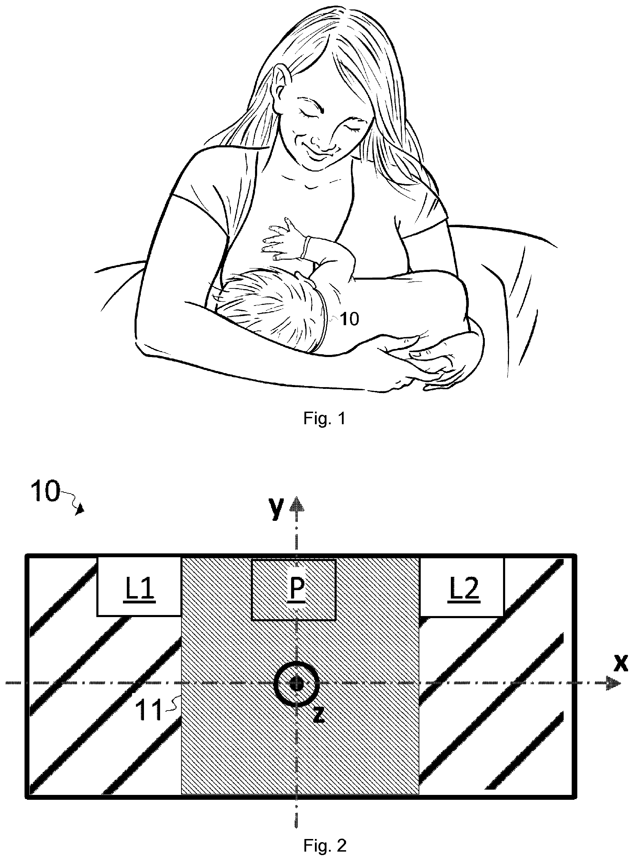

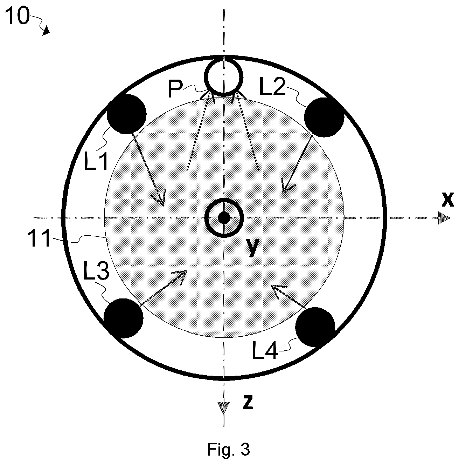

Figure 1 illustrates domestic use of the collar (10) depicted in Figure 2 and Figure 3. Comparable to a pulse oximeter, it measures the flow rate or amount of milk imbibed percutaneously at the infant's neck (11) based on differences in optical interference. To this end, upon transillumination of the neck (11) at specific wavelengths, the mass or molar attenuation coefficient of breast milk, which is perceived as white to yellow, may be used to distinguish such fluid from other bodily chemical species.

For this purpose, four LEDs (L1, L2, L3, L4) or laser diodes serve as optical excitation sources that are activated sequentially. Absorption of the emitted light within the neck (11) is translated into electric current using a photodiode (P) as a receiver. To minimize external interference, the periphery of the transmitters (L1, L2, L3, L4) and receiver (P) is screened off by the shielding collar (10).

For correcting any superficial variance, such as in terms of thickness of the skin tissue, a relative rather than an absolute measure is applied. Hence, in a preliminary step, the maximum and minimum flow rate achievable by an infant is related to light intensity as a function of the attainable wavelengths. Hereinafter, this measure shall be referred to as the fractional milk flow rate. Such fractional flow rate will be proportional to the total flow rate.

An established ratio between fractional and total flow rate may be reflected constructively in the photodiode's gain such that the electrical current output by the photodiode (P) relates directly to the total flow rate, although such relation should not be considered essential to the method at hand. Alternatively, total flow rate can be retrieved from a lookup table.

Moreover, a transceiver having an antenna for transmission and reception may be provided for communications, such as via a cellular network, WLAN, WPAN, or by cable. Suitable transmission protocols include TCP/IP. It is well understood that any suitable transmission medium and protocol may be used for this purpose. Communications may be compressed as required.

For optimal approximation of effective milk intake, various numbers and combinations of diodes (L1-L4, P) with regard to wavelength, arrangement, and scattering angle are conceivable without departing from the scope of the present invention. Likewise, a computerized assistant, including but not limited to a smartphone application, may be provided to evaluate and optimize the nursing routine.

### Industrial applicability

The invention is applicable, inter alia, throughout the medical equipment industry.

### Reference signs list

| | |
|---|---|
| 10 | Collar |
| 11 | Infant's neck |
| L1 | White LED |
| L2 | Yellow LED |
| L3 | Red LED |
| L4 | Infrared LED |
| P | Photodiode |

### Citation list

The following documents are cited hereinbefore.

### Patent literature

PTL1: US 1644257 A (EDWARD LASKER) 10.04.1927
PTL 2: CN 110338757 A (HANGZHOU CHILDRENS HOSPITAL) 18.10.2019
PTL3: WO 01/54488 A (VASLOV TRADERS PROPRIETARY LTD) 02.08.2001
PTL4: US 2019125244 A (MOLKENTHIN BRITTANY) 02.05.2019

### Non-patent literature

NPL1: **NEIFERT,** Marianne, et al. Overcoming clinical barriers to exclusive breastfeeding. Pediatric Clinics. 2013, vol.60, no.1, p. 115-145.

## Claims

1. Method of monitoring intake of breast milk by an infant wearing a collar (10), comprising
calculating the intake, for example, in terms of volume, mass, or energy, from a flow rate of the milk through a cross-sectional area of the infant's esophagus,
**characterized in**
transmitting light from light sources (L1-L4) through the esophagus, receiving the transmitted light at photosensors (P) arranged diametrically opposite the light sources (L1-L4) with respect to the esophagus, and, based on the light received, determining the intake by estimating the flow rate by means of absorption spectroscopy.

2. Method as per Claim 1 wherein the flow rate is estimated by comparing intensity of the light received at the photosensors (P) with predetermined reference intensities associated with a minimum and maximum flow rate.

3. Method as per Claim 2 wherein the estimated flow rate is retrieved from a lookup table based on the intensity of the light received.

4. Method as per Claim 1 or Claim 2 wherein photocurrent through the photosensors (P) is amplified into an output current linearly dependent on the flow rate.

5. Method as per any of the preceding claims wherein the light is visible or infrared.

6. Method as per any of the preceding claims wherein three among the light sources (L1-L4) emit monochromatically at wavelengths of 575 nm, 700 nm, and 910 nm, respectively.

7. Method as per Claim 5 or Claim 6 wherein one among the light sources (L1-L4) emits white light.

8. Method as per any of the preceding claims wherein the light sources (L1-L4) are operated alternately, preferably sequentially.

9. Collar (10), preferably opaque to ambient light, having light sources (L1-L4) diametrically opposed to photosensors (P) and means adapted to execute the steps of the method of any of the preceding claims.

10. Collar as per Claim 9 wherein the light sources (L1-L4) and photosensors (P) are semiconductor diodes.

11. Collar as per Claim 10 wherein the light sources (L1-L4) are laser or light-emitting diodes (L1-L4).

12. Computer program comprising instructions to execute the steps of the method of any of Claim 1 through Claim 8 when run on a processor connected to the collar (10) of any of Claim 9 through Claim 11.

13. Data carrier signal carrying the computer program of Claim 12.

## Patentansprüche

1. Verfahren zur Überwachung der Aufnahme von Muttermilch durch
einen Säugling, der ein Halsband (10) trägt, umfassend
Berechnen der Aufnahme, zum Beispiel in Form von Volumen, Masse oder Energie, aus einer Fließgeschwindigkeit der Milch durch eine Querschnittsfläche der Speiseröhre des Säuglings
**gekennzeichnet durch**
Aussenden von Licht aus Lichtquellen (L1-L4) durch die Speiseröhre, Empfangen des übertragenen Lichts an Fotosensoren (P), die bezüglich der Speiseröhre diametral gegenüber den Lichtquellen (L1-L4) angeordnet sind, und
Bestimmen der Aufnahme anhand des empfangenen Lichts durch Abschätzen des Volumenstroms mittels Absorptionsspektroskopie.

2. Verfahren nach Anspruch 1, wobei
der Volumenstrom durch Vergleich der Intensität des an den Fotosensoren (P) empfangenen Lichts mit vorgegebenen Referenzintensitäten geschätzt wird, die einem minimalen und maximalen Volumenstrom entsprechen.

3. Verfahren nach Anspruch 2, wobei
der geschätzte Volumenstrom anhand der Intensität des empfangenen Lichts aus einer Umsetzungstabelle abgerufen wird.

4. Verfahren nach Anspruch 1 oder 2, wobei
der durch die Fotosensoren (P) fließende Fotostrom zu einem linear vom Volumenstrom abhängigen Ausgangsstrom verstärkt wird.

5. Verfahren nach einem der vorhergehenden Ansprüche, wobei
das Licht sichtbar oder infrarot ist.

6. Verfahren nach einem der vorhergehenden Ansprüche, wobei
drei der Lichtquellen (L1-L4) monochromatisch bei Wellenlängen von 575 nm, 700 nm bzw. 910 nm emittieren.

7. Verfahren nach Anspruch 5 oder 6, wobei
eine der Lichtquellen (L1-L4) weißes Licht emittiert.

8. Verfahren nach einem der vorhergehenden Ansprüche, wobei
die Lichtquellen (L1-L4) wechselweise, vorzugsweise der Reihe nach, betrieben werden.

9. Halsband (10), vorzugsweise undurchlässig für Umgebungslicht, mit Lichtquellen (L1-L4), die Fotosensoren (P) diametral gegenüberliegen, und Mitteln, die geeignet sind, die Schritte des Verfahrens nach einem der vorhergehenden Ansprüche auszuführen.

10. Halsband nach Anspruch 9, wobei
die Lichtquellen (L1-L4) und Fotosensoren (P) Halbleiterdioden sind.

11. Halsband nach Anspruch 10, wobei
die Lichtquellen (L1-L4) Laser oder Leuchtdioden (L1-L4) sind.

12. Computerprogramm mit Befehlen zum Ausführen der Schritte des Verfahrens nach einem der Ansprüche 1 bis 8, wenn es auf einem Prozessor ausgeführt wird, der mit dem Halsband (10) nach einem der Ansprüche 9 bis 11 verbunden ist.

13. Datenträgersignal, das das Computerprogramm nach Anspruch 12 überträgt.

## Revendications

1. Méthode de surveillance de la consommation de lait maternel
par un nourrisson portant un collier (10), comprenant
calculer la consommation, par exemple, en termes de volume, masse ou énergie, à partir d'un débit du lait à travers une section transversale de l'œsophage du nourrisson,
**caractérisé par**
transmettre de la lumière à partir de sources lumineuses (L1-L4) à travers l'œsophage,
recevoir la lumière transmise à des photocapteurs (P) disposés diamétralement opposés aux sources lumineuses (L1-L4) par rapport à l'œsophage et,
à partir de la lumière reçue, déterminer la consommation en estimant le débit au moyen de la spectroscopie d'absorption.

2. Méthode selon la revendication 1 dans laquelle
le débit est estimé en comparant l'intensité de la lumière reçue aux photocapteurs (P) avec des intensités de référence prédéterminées associées à un débit minimal et maximal.

3. Méthode selon la revendication 2 dans laquelle
le débit estimé est extrait d'une table de consultation en fonction de l'intensité de la lumière reçue.

4. Méthode selon la revendication 1 ou 2 dans laquelle
le photocourant traversant les photocapteurs (P) est amplifié en un courant de sortie dépendant linéairement du débit.

5. Méthode selon l'une quelconque des revendications
précédentes dans laquelle
la lumière est visible ou infrarouge.

6. Méthode selon l'une quelconque des revendications
précédentes dans laquelle
trois des sources lumineuses (L1-L4) émettent de manière monochromatique à des longueurs d'onde de 575 nm, 700 nm et 910 nm, respectivement.

7. Méthode selon la revendication 5 ou 6 dans laquelle
l'une des sources lumineuses (L1-L4) émet de la lumière blanche.

8. Méthode selon l'une quelconque des revendications
précédentes dans laquelle
les sources lumineuses (L1-L4) sont actionnées alternativement, de préférence séquentiellement.

9. Collier (10), de préférence opaque à la lumière ambiante, comprenant des sources lumineuses (L1-L4) diamétralement opposées à des photocapteurs (P) et des moyens adaptés pour mettre en oeuvre les étapes de la méthode de l'une quelconque des revendications précédentes.

10. Collier selon la revendication 9 dans lequel
les sources lumineuses (L1-L4) et les photocapteurs (P) sont des diodes semiconductrices.

11. Collier selon la revendication 10 dans lequel
les sources de lumière (L1-L4) sont des lasers ou des diodes électroluminescentes (L1-L4).

12. Programme d'ordinateur comprenant des instructions pour mettre en oeuvre les étapes de la méthode de l'une quelconque des revendications 1 à 8 lorsque le programme est exécuté par un processeur connecté au collier (10) de l'une quelconque des revendications 9 à 11.

13. Signal d'un support de données, portant le programme d'ordinateur selon la revendication 12.
